# FASCICULE DE BREVET EUROPEEN

(11) **EP 0 589 943 B1**
(45) Date de publication et mention de la délivrance du brevet: **13.03.2002**
(21) Numéro de dépôt: 92911693.7
(22) Date de dépôt: 05.06.1992
(51) Int. Cl.: C07K 14/35, C07K 16/12, G01N 33/68, A61K 39/04

(54) **PROTEINES DE MYCOBACTERIUM ET APPLICATIONS**
MYCOBACTERIUMPROTEIN UND DESSEN VERWENDUNG
MYCOBACTERIUM PROTEINS AND APPLICATIONS

(30) Priorité: 07.06.1991 FR 9106970
(43) Date de publication de la demande: 06.04.1994
(73) Titulaire: INSTITUT PASTEUR, 75724 Paris Cédex 15 (FR)
(72) Inventeur: MARCHAL, Gilles, F-94200 Ivry (FR); ROMAIN, Félix, F-91640 Fontenay-les-Briis (FR); PESCHER, Pascale, F-75018 Paris (FR)
(74) Mandataire: Phélip, Bruno
(86) Numéro de dépôt international: FR9200508
(87) Numéro de publication internationale: WO9221758

(56) Documents cités:
- WO-A-89/09261
- INFECTION AND IMMUNITY vol. 55, no. 12, Décembre 1987, WASHINGTON DC, USA pages 3213 - 3214; C. ABOU-ZEID ET AL.: 'Characterization of the secreted antigens of Mycobacterium bovis BCG: Comparison of the 46-kilodalton dimeric protein with proteins MPB64 and MPB70.'

## Description

La présente invention a pour objet des protéines de Mycobactérium en particulier de M. bovis, ayant des poids moléculaires compris entre environ 44,5 et 47,5 kD et les séquences nucléotidiques codant pour ces protéines .

La présente invention a aussi pour objet des fractions protéiques obtenues à partir de cultures de Mycobactérium bovis, présentant une réactivité immunologique spécifique vis-à-vis d'anticorps anti-tuberculose.

Elle est également relative à l'utilisation de ces protéines et fractions pour la détection et le suivi de la tuberculose ainsi que comme vaccin.

La tuberculose continue d'être un problème de santé publique dans le monde. Le nombre des décès annuel directement en rapport avec la tuberculose est d'environ 3 millions et le nombre des nouveaux cas de tuberculose est d'environ 15 millions. Ce nombre de décès dus à la tuberculose est encore élevé pour les pays développés; par exemple pour la France, il est de l'ordre de 1500 par an, chiffre certainement sous-évalué d'un facteur 2 ou 3 si l'on considère les évaluations de Roujeau sur les discordances entre les chiffres officiels et les résultats d'autopsies systématiques. La récente augmentation des cas de tuberculose, ou du moins l'arrêt de la décroissance de la fréquence de cette maladie, en corrélation avec le développement de l'épidémie de VIH/SIDA, est à prendre en compte. Au total la tuberculose reste la première maladie infectieuse par sa fréquence pour la France et les pays développés, mais surtout pour les pays en voie de développement pour lesquels elle constitue la source principale de pertes humaines en rapport avec une seule maladie.

Actuellement, le diagnostic de certitude apporté par la mise en évidence de bacilles cultivables dans un prélèvement provenant du malade n'est obtenu que pour moins de la moitié des cas de tuberculose. Même dans les cas de tuberculose pulmonaire qui représente 80 à 90% des atteints de tuberculose et qui est la forme de la maladie pour laquelle la mise en évidence de bacilles est la plus facile, l'examen des expectorations n'est positif que dans moins de la moitié des cas.

Le développement des techniques les plus sensibles, telles que la PCR (Réaction par Polymérisation de Chaîne), se heurte toujours à la nécessité d'obtenir un prélèvement . Les femmes et les enfants ne crachant habituellement pas, les prélèvements pour les formes de l'enfance nécessitent souvent une intervention médicale relativement spécialisée (biopsie ganglionnaire ou prélèvement par ponction lombaire du liquide céphalo-rachidien par exemple) .

Par ailleurs, des inhibitions de la réaction PCR elle-même existent, de sorte qu'un prélèvement peut être inexploitable par cette technique du fait de l'impossibilité de contrôler leurs origines.

Enfin, le diagnostic bactériologique classique, examen microscopique et culture, par sa limite de sensibilité (au mieux de l'ordre de 10⁴ à 10⁵ bacilles dans le prélèvement) suppose qu'il y ait déjà eu un développement relativement important des bacilles et donc de la maladie.

La mise en évidence d'anticorps spécifiques dirigés contre Mycobacterium tuberculosis pourrait donc apporter une aide pour le diagnostic des formes fréquentes de la maladie pour lesquelles la mise en évidence des bacilles eux-mêmes est difficile ou impossible

Des générations successives de chercheurs ont tenté de mettre au point une technique de diagnostic sérologique de la tuberculose. De Ardoing (C.r. hebd. Séanc. Acad. Sci. Paris; 1898; 126: 1398-1401) à Middlebrook et Dubos (J. Exp. Med. 1948, 88: 521-528), les préparations utilisées pour ce diagnostic ont été très peu ou pas purifiées, l'effort portant surtout sur un accroissement de la sensibilité et non de la spécificité. Récemment encore des techniques tendant à accroître seulement la sensibilité ont été proposées en utilisant soit une technique de type ELISA soit une technique de type RIA.

Les travaux plus récents de Daniel et Janicki (Microbiol. Rev. 1978, 42: 84-113) ou Wiker et al. (Scand. J. Immunol. 1988, 27: 223-239), ont montré la complexité des antigènes de mycobactéries. A partir de ceux-ci, on a tenté de définir des antigènes principaux pouvant être utilisés dans un but de diagnostic (Chan et al. Am. Rev. Respir. Dis. 1990, 142: 385-390). Un test sérologique est ainsi commercialisé (ANDA) depuis 3 ou 4 ans. Il utilise un antigène très complexe, mal caractérisé biochimiquement l'A60 de Cocito et Vanlinden (Clin. Exp. Immunol.1986, 66: 262-272). La spécificité et la sensibilité de ce test sont médiocres. Il n'apporte pas une grande aide pour le diagnostic et est très critiqué voire rejeté par de nombreux biologistes qui l'ont utilisé de façon large.

Des antigènes bien caractérisés, correspondant à des protéines abondantes quantitativement, ont été aussi testés pour leur spécificité et leur sensibilité pour le diagnostic sérologique.

L'antigène a d'environ 35 kd a été purifié dès 1965; il représente 25 à 35 % des protéines présentes dans le milieu de culture de nombreuses souches de M.tuberculosis, M. kansasii ou BCG, (Fukui et al. Biken J.1965, 8: 189-199).

Un antigène de 64 kd, représentant plus de 50% des protéines des milieux de cultures, lorsque ces dernières sont effectuées en l'absence de zinc, a aussi été purifié (De Bruyn et al. J. Gen. Microbiol. 1981, 124: 353-357).

Une seconde molécule, représentant 25 à 30% des protéines dans les mêmes milieux, a également été purifiée. Cette molécule de 32 kd est très proche de l'antigène a déjà décrit, mais est néanmoins différente (De Bruyn et al. Microbiol. Pathogenesis 1987, 2 : 351-366).

De même, une molécule de 16 kD a été purifiée, et l'on a constaté sa présence dans le milieu de culture de certaines souches de BCG (NCG Tokyo) et M. bovis et son absence ou son faible niveau pour d'autres souches (BCG Copenhague, Pasteur) (Harboe et Nagai, Am. Rev. Respir. Dis. 1984, 129 :444-452).

ABOU ZEID et al. (Infection and Immunity, 1987, vol.55 (12): 3213-3214) ont caractérisé une protéine dimérique de 46 kD secrétée par *Mycobacterium bovis* BCG, composée de deux monomères de 23 kD observables sur un gel d'électrophorèse en conditions dénaturantes.

La demande PCT n° WO 89/09 261 décrit une méthode de diagnostic d'une infection par *Mycobacterium bovis* mettant en oeuvre des anticorps dirigés contre la protéine MPB-70 de *M*. *bovis,* qui constitue un antigène majeur de cette bactérie retrouvée à raison de plus de 10% du contenu total en protéine présent dans des filtrats de culture de ces organismes.

Les critères de sélection des protéines utilisées par ces auteurs ont donc été en premier lieu des critères biochimiques. Les protéines n'ont été testées que dans un second temps pour leur capacité à détecter l'infection par la tuberculose.

Des résultats représentatifs de cette démarche montrent ainsi que la réponse en anticorps dirigés contre la protéine de choc thermique de 64 kD mycobactéries est élevée chez 80% des malades atteints de tuberculose, mais aussi chez 30% des enfants atteints de coqueluche (Thole et al. Infect. Immun. 1987, 55 : 1466-1475).

L'utilisation des techniques de biologie moléculaire pour cloner un gène dont le produit est reconnu par un anticorps polyclonal de spécificité restreinte pu un anticorps monoclonal, a conduit sensiblement aux mêmes résultats. Dans ce cas, les anticorps qui ont été utilisés comme sondes, ont été préparés contre des antigènes présents dans des extraits de bactéries ou sur des bactéries tuées. Ces extraits ou bactéries ayant été injectés en réglé générale à des souris Balb/C, le répertoire des réponses obtenues est limité à la réponse de souris de cette seule lignée, soumise de plus à des protocoles expérimentaux très semblables.

Les molécules, ayant été ainsi reconnues par certains anticorps monoclonaux, comme la 65 kD ou la 32 kD, ont déjà été purifiées ou sont en cours de purification par l'approche biochimique classique.

De nouvelles molécules comme la 70 kD ou la 19 kD, ont été aussi mises en évidence par ces techniques. Mais lorsqu'elles sont utilisées dans un but de détection, ces molécules ne permettent pas de distinguer de façon non ambiguë les sujets malades atteints de tuberculose des sujets normaux ou présentant d'autres maladies infectieuses.

Un travail récent a tenté de purifier des antigènes de mycobactéries en utilisant un mélange de sérums de malades tuberculeux pour fabriquer un immuno adsorbant permettant de purifier partiellement les antigènes principaux reconnus par les malades (Thongkrajai et al. J. Med. Microbiol. 1989, 30: 101-104).

Les techniques rapportées dans l'art antérieur sont donc majoritairement basées sur l'isolement préliminaire de protéines sur leurs caractères biochimiques. Ce n'est qu'après cet isolement que les auteurs testent la capacité de ces protéines à détecter les individus atteints par la tuberculose.

Dans les travaux ayant conduit à la présente invention, on a choisi une autre méthode pour sélectionner des antigènes représentatifs de l'infection tuberculeuse.

Selon l'invention, on s'est attaché à sélectionner des antigènes représentatifs sans ambiguïté de l'infection tuberculeuse à l'aide de sêrums provenant de malades atteints de tuberculose ou de cobayes immunisés par des bacilles vivants.

Cette méthode qui se démarque des expérimentations décrites dans l'art antérieur, a permis l'isolement d'antigènes représentatifs de la tuberculose, permettant la détection sans ambiguïté des malades atteints par cette maladie.

La présente invention a donc pour objet une fraction protéique de mycobactérium et en particulier de M. bovis présentant un poids moléculaire compris entre 44,5 et 47,5 kD environ. Ces protéines peuvent posséder des poids moléculaires d'environ 45 kD ou d'environ 47 kD, avec des intervalles d'erreur de +/- 10%, et des pH isoélectriques (pHi) d'environ 3,7 (protéines 45 et 47 kP) et de 3,9 (protéines de 47 kd), avec des intervalles d'erreur de pHI de +/- 0,2.

L'erreur de 10% dans la détermination des poids moléculaires est notamment imputable aux variations de résultats en fonction des kits de détermination utilisés.

Cette fraction protéique possède en outre une composition en acides aminés exprimée en fréquence pour PRO d'environ 21,9% , pour ASN/ASP d'environ 10,6% , pour THR d'environ 5,4%, pour SER d'environ 5% , pour GLN/GLU d'environ 6% ,pour GLY d'environ 7,4% , pour ALA d'environ 19,2%, pour VAL d'environ 5,8 %, pour ILE d'environ 2,3% , pour LEU d'environ 4,7% , pour TYR d'environ 2,2% , pour PHE d'environ 2,2% , pour LYS d'environ 2,9% , et/ou pour ARG d'environ 2,5%.

L'espèce protéique de 47 kD peut avoir une extrémité NH₂ terminale ayant la séquence suivante:

ALA-PRO-GLU-PRO-ALA-PRO-PRO-VAL-PRO-PRO-ALA-ALA-ALA-ALA-
 1   2   3   4   5   6   7   8   9  10  11  12  13  14
PRO-PRO-ALA
 15  16  17

La présente invention a d'autre part pour objet une lignée d'hybridomes déposée le 12 Avril 1991. sous le n°I.1081 auprès de la Collection Nationale de Culture des Microorganismes ( CNCM ) de l'Institut Pasteur , et les anticorps sécretés par cette lignée.

Les protéines précédemment décrites présentent aussi la caractéristique d'être reconnues par des anticorps présents dans le sérum de patients atteints par la tuberculose ou d'animaux susceptibles d'être atteints par la tuberculose , par des anticorps obtenus par immunisation avec des bacilles de M.bovis vivants , ou par un anticorps sécrété par la lignée d'hybridomes n°I.1081 précitée, et de n'être pas reconnues par des anticorps obtenus par immunisation de cobayes avec des bacilles de M.bovis tués par la chaleur ou par des anticorps de patients sains ou atteints par une maladie autre que la tuberculose .

Ces protéines sont aussi caractérisées par le fait qu'elles peuvent être présentes dans le milieu de culture .

Selon un mode de mise en oeuvre particulier de l'invention , un épitope provenant d'un agent biologique autre que M. bovis peut aussi être greffé sur une des protéines précédemment définies.

On obtient ainsi des protéines hybrides comprenant une des proteines précédemment décrites et un déterminant antigénique provenant d'un agent biologique autre que M.bovis.

Ce déterminant peut être de diverses natures et notamment être un fragment d'antigène protéique ou glycoprotéique, en vue d'obtenir des compositions immunogènes susceptibles d'induire la synthèse d'anticorps dirigés contre ces épitopes multiples .

L'utilisation d'agents de pontage bifonctionnels tels que le glutaraldéhyde ou la benzoquinone ou le N-bromo succinimide bien connus pour leurs propriétés de couplage de protéines entre elles, ou tels que l'hydrazide permettant le couplage de résidus glycosylés avec des protéines , peuvent être utilisés pour la formation de molécules hybrides . Ces molécules hybrides peuvent être constituées en partie d'une molécule porteuse (complexe 45-47 kD) associée à un ou plusieurs épitopes ou fragments d'antigènes par exemple la toxine diphtérique ou des fragments de celle-ci, la toxine tétanique, l'antigène HBS du virus HBV , l'antigène VP1 du virus de la poliomyélite .

De telles protéines peuvent ainsi induire l'immunisation contre des protéines ou fragments de protéines correspondant à des déterminants antigéniques n'étant pas présents sur les protéines de M.bovis.

La présente invention est en outre relative à des fractions protéiques obtenues à partir de cultures de Mycobactérium et en particulier de M.bovis par un procédé comprenant au moins les étapes suivantes :
- élimination des bactéries du milieu de culture par filtration,
- passage du filtrat sur tamis moléculaire et répartition de l'éluat en fractions , et
- sélection des fractions par détermination de leur réactivité vis-à-vis d'anticorps spécifiques et de la tuberculose.

Les fractions obtenues par filtration sur tamis moléculaire peuvent aussi être soumises à une chromatographie échangeuse d'ions et optionnellement à une chromatographie en phase inverse .

La présente invention a d'autre part pour objet l'application des protéines ou fractions protéiques ou anticorps tels que précédemment définis pour la détection et le suivi de la tuberculose en particulier chez l'homme et les bovins . Une telle détection peut notamment être mise en oeuvre par la méthode du Western Blot (immunoempreinte) ou une méthode immunoenzymologique (ELISA) ou par une méthode radioimmunologique (RIA), à l'aide d'un coffret ou kit de dosage , contenant ces protéines ainsi que notamment des tampons permettant d'effectuer la réaction immunologique et en outre des substances permettant de la révéler .

La présente invention est en outre relative à des vaccins ou médicaments contenant au moins une protéine , une fraction protéique ou un anticorps tels que précédemment définis .

Des vaccins contenant les protéines non greffées pourront être utilisés pour immuniser des individus à l'encontre de la tuberculose . Les protéines portant un épitope provenant d'un agent biologique autre que M.bovis peuvent être utilisés dans le cadre d'immunisations contre d'autres maladies .

A titre indicatif , on pourra utiliser de 50 à 500µg de protéine pour la dose d'un individu , ou de 10⁵ à 10⁶ bactéries recombinantes / individu par voie intradermique.

La présente invention a encore pour objet une composition pharmaceutique contenant au moins une quantité pharmaceutiquement efficace d'une protéine , fraction protéique ou d'un anticorps tel que précédemment défini en association avec des diluants ou des adjuvants pharmaceutiquement compatibles .

Sous un autre aspect, la présente invention est relative à l'utilisation des protéines , fractions protéines ou anticorps tels que précédemment définis pour la fabrication d'un médicament pour le traitement ou la prévention de la tuberculose .

La présente invention est illustrée sans pour autant être limitée par les exemples de mise en oeuvre suivants et en référence aux dessins annexés dans lesquels :
La figure 1 représente le profil de densité optique (D0) à 220 et à 280 nanomètres de la filtration moléculaire (Si 300) du milieu de culture de M.bovis.
La figure 2 représente le profil en densité optique à 220 nanomètres de la séparation sur colonne échangeuse d'ions (DEAE ) des molécules provenant de la fraction 2 obtenue lors de la filtration moléculaire précédente .
La figure 3 est le profil de densité optique à 220 nanomètres de la chromatographie sur colonne en phase inverse de la fraction 1 issue de la chromatographie échangeuse d'ions.
Les figures 4A et 4B sont des photographies des membranes de PVDF mises en présence respectivement d'un mélange de sérums de cobayes immunisés avec des bacilles morts (A) ou vivants ( B ) .
La figure 4C est un gel d'électrophorèse après coloration par du bleu de Coomassie du matériel de départ (O) et des fractions obtenues sur tamis moléculaire (1 à 6 ) . Des gels identiques ont été transférés sur membrane de PVDF et révélés par des sérums de cobayes immunisés par des bacilles morts (4A) ou vivants (4B )
Les figures 5A et 5B représentent des membranes de PVDF correspondant à un gel obtenu par migration des fractions obtenues sur colonne échangeuse d'ions (1 à 3) et de la fraction 2 obtenue par filtration sur tamis moléculaire, ladite membrane étant mise en présence d'anticorps de sérums de cobayes immunisés respectivement avec des bacilles morts (A) ou vivants (B). La figure 5C représente le gel à l'origine des transferts des figures 5A et 5B, coloré par le bleu de Coomassie.
Les figures 6A et 6B représentent l'empreinte des gels sur des membranes correspondant à la migration de la fraction 1 obtenue sur colonne échangeuse d'ions (0) et des fractions obtenues par chromatographie en phase inverse (1 à 5), mises en présence des anticorps des sérums de cobayes immunisés avec respectivement les bacilles morts (6A) et vivants (6B). Il est à noter que dans la fraction 1 de cette étape de purification, il existe une contamination par du matériel de départ due à un chargement trop important de la colonne de chromatographie en phase inverse.
Les figures 7A à 7L représentent des empreintes des gels sur des membranes obtenus à partir de l'électrophorèse de matériel de départ (0), des fractions obtenues par filtration sur tamis moléculaire (1 à 6) et mises en présence de sérums individuels provenant de malades atteints de tuberculose (7A à 7F) correspondant respectivement au sérum des malades (N° 77, N° 115, N° 117, N° 108, N° 104, N° 105) ou par des sérums individuels provenant de malades atteints de borréliose (7G à 7K) ou de yersiniose (7L).

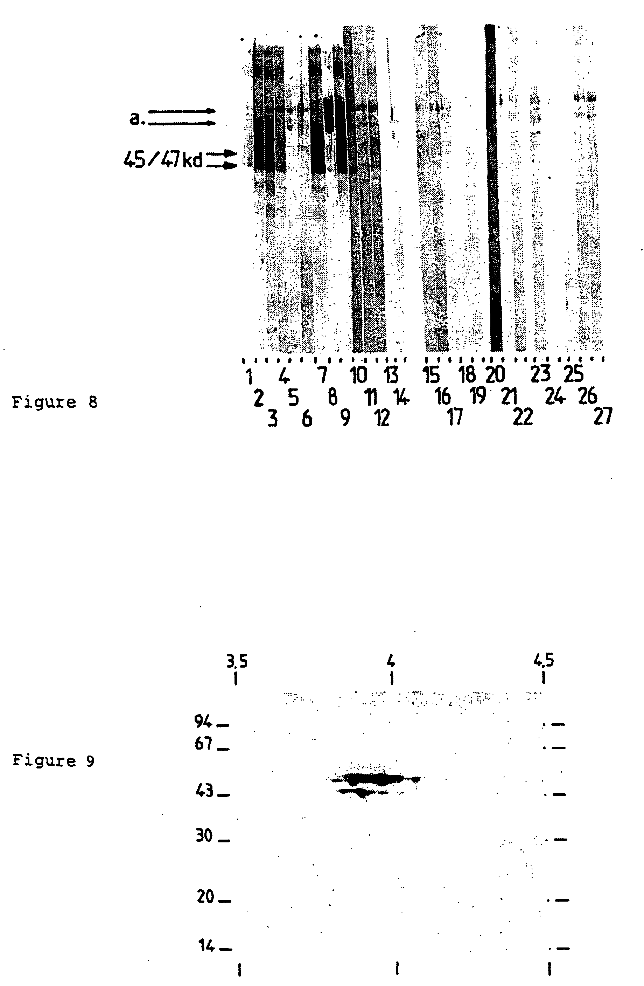

La figure 8 représente une empreinte d'un gel sur une membrane de PVDF correspondant à une électrophorèse de la fraction 5 obtenue en chromatographie en phase inverse. Cette membrane a été coupée en bandelettes de 3 mm environ et chaque bandelette a été mise en contact d'un sérum individuel ( dilué au 1/20) d'un malade atteint de tuberculose ( bandes 1 à 14 correspondant respectivement aux malades N° 77 , 104, 105, 108, 115, 117 , 124, 131, 134, 123, 3a, 2g , 2d , et 2a ) ou de malades atteints de borréliose ( bandes 15 à 19 ) de leptospirose ( bandes 20 à 22 ) de yersiniose ( bandes 23 et 24) ou de brucellose ( bandes 25 à 27 ). La double marque " a" correspond à un artéfact habituel dans cette technique .
La figure 9 est une photographie d'un gel en deux dimensions coloré au nitrate d'argent , des protéines de 45-47 kD, ( fraction 5 ) .

### EXEMPLE 1 :

### Procédé de purification des antigènes

### 1) Obtention des antigènes :

Des cultures de BCG ( souche 1173 P2 ) sont effectuées en ballons contenant 130 ml de milieu synthétique de Sauton selon une technique classique ( Gheorghiu et al. Bull. Institut Pasteur 1983, 81 : 281-288 ) . Le milieu de culture est récolté après 14 jours à 37°, décanté et filtré ( 0,22 µm) à 4°C . Placé sur une membrane Amicon (PM10) sous une pression d'azote de 2 bars et toujours à 4°C , le milieu de culture est lavé intensivement avec de l'eau rétro-osmosée contenant 4% de butanol , puis concentré 10 à 20 fois par rapport au volume initial . Ce milieu de culture concentré , contenant les molécules non exclues par la membrane PM10 Amicon, est lyophilisé, pesé et conservé sous forme de poudre à -20°C . Les 12g du matériel de départ utilisés pour le schéma de purification décrit ci-dessous sont obtenus à partir de 30 litres de milieu de culture .

### Schéma de purification :

### 2) Gel filtration:

Une colonne préparative Si300, 3 µm de 50 x 750 mm (SERVA), est équilibrée par passage d'une solution saline tamponnée (Na₂HPO₄ 50 mM ajustée à pH 7,5 avec KH₂PO₄) contenant 4% de butanol ; cette solution est préalablement filtrée sur une membrane ( 0,22µm ) . Le débit de la colonne est ajusté à 1,25 ml par mn; la pression maximum réglée à 45 bars n'est pas atteinte .

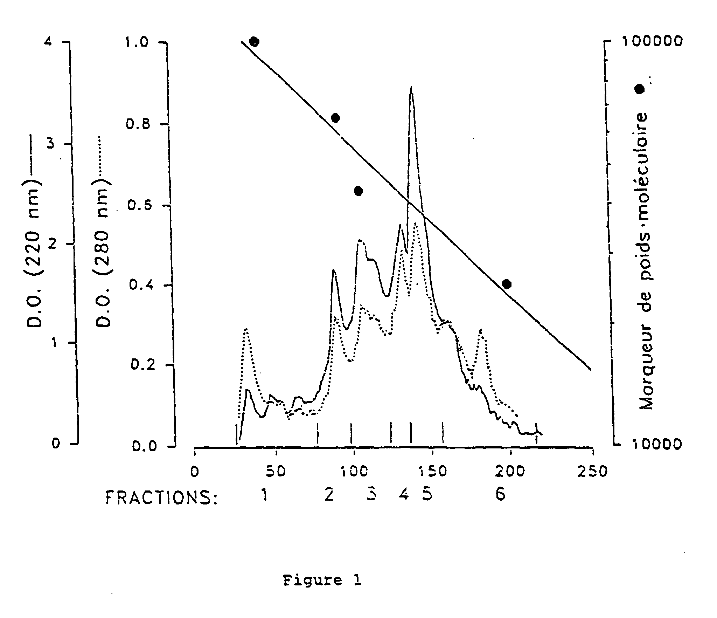

Le matériel à injecter sur la colonne est préparé à la concentration de 50mg/ml dans la solution tampon/butanol, ultracentrifugé à 40 000 g durant 2 h puis filtré sur membrane (0,22 µm) . Des échantillons de 10 ml sont préparés et congelés à -20°C . Chaque échantillon de 10 ml, filtré de nouveau après décongélation et injecté sur la colonne , contient environ 500 mg de matériel brut . Les profils des densités optiques à 280 et 220 nm sont rapportés figure 1 pour une séquence typique de séparation . Les six fractions principales choisies d'après le profil sont concentrées à 4°C et lavées intensivement sur membrane Amicon PM10 avec de l'eau rétro-osmosée contenant 4% de butanol . Chaque fraction concentrée est lyophilisée, pesée puis conservée à -20°C . La fraction 2 de cette étape contient les molécules principales reconnues par les anticorps des cobayes immunisés avec des bacilles vivants ou par les anticorps des malades tuberculeux. Seule cette fraction est soumise à l'étape suivante .

### 3) Colonne échanqeuse d'ions :

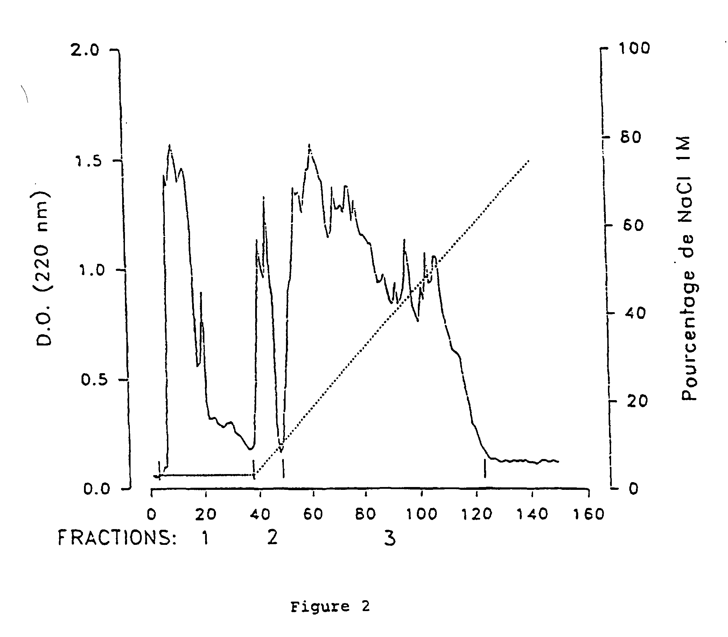

Une colonne préparative DEAE-TSK 5PW de 21,5 x 150 mm (LKB) est équilibrée avec une solution saline tamponnée (Na₂HPO₄/ NaH₂PO₄ 10 mM , pH = 7,5 et NaCl 10 mM) contenant 4% de butanol . La pression maximum est inférieure à 30 bars pour un débit de 6 ml/mn. Pour le tampon d'élution , seule la concentration de NaCl est modifiée (1 M) . Un gradient linéaire est appliqué selon le schéma indiqué figure 2 après injection d'un volume d'échantillon de 4 ml contenant au total 100 mg du matériel précédent . Les fractions principales sont recueillies selon le profil en densité optique à 220 nm. Ces fractions sont concentrées et lavées sur membrane PM₁₀ (Amicon) par de l'eau rétro-osmosée contenant 4% de butanol, puis lyophilisées . Une fois pesée, chaque fraction est conservée à -20°C . Seule la fraction 1 de cette étape contient la majorité des molécules reconnues par les anticorps des cobayes immunisés avec des bacilles vivants ; elle est soumise à l'étape suivante de séparation .

### 4 ) Colonne phase inverse :

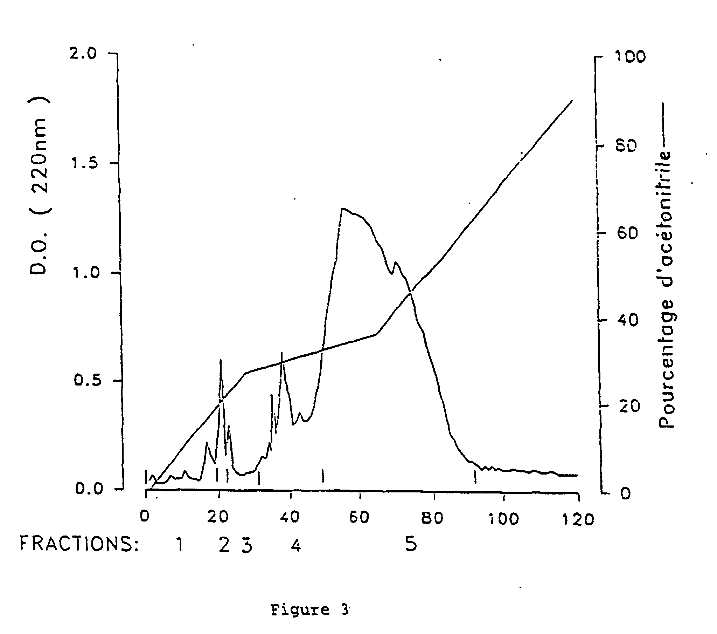

Une colonne RP 300 C₈ 10 µm de 4,6 x 250 mm (Aquapore Brownlee lab.) est équilibrée avec un tampon acétate d'ammonium (NH₄COO CH₃ 20 mM) filtré sur 0,22 µm avec un débit de 2 ml/mn sous une pression maximum de 115 bars . Le tampon d'élution contenant 90% d'acétonitrile est appliqué selon le profil indiqué sur la figure 3 après injection d'un échantillon de 10 mg sous un volume de 1 ml. Le profil de densité optique à 220 nm permet de séparer cinq fractions principales qui sont concentrées par évaporation sous vide à 40°C , puis lyophilisées .

### 5 ) Immunodétection des antigènes :

Des gels dénaturants à 10 % de polyacrylamide , 0,1% de SDS sont préparés selon la technique classique de Laemmli , (Nature 1970, 277 : 680-685 ). Des échantillons contenant entre 10 et 2 µg de matériel , en fonction de l'étape de la purification, sont appliqués dans un tampon contenant 5% de mercaptoéthanol, 3% de SDS et une trace de bleu de bromophénol sous un volume de 10 µl dans chaque piste du gel. Après électrophorèse jusqu'à la limite de migration du bleu, les molécules présentes dans les échantillons sont transférées sur une feuille de PVDF (Millipore) en appliquant un champ électrique modéré durant une nuit (Harlow et Lane, Antibodies,A Laboratory manual. Cold Spring Harbor Laboratory (eds) 1988 ) .

Une coloration de la feuille de PVDF par une solution de bleu de Coomassie durant moins d'une minute, suivie d'une décoloration, permet le repérage des marqueurs de poids moléculaires dont les contours sont entourés d'un trait de crayon. Après décoloration totale , la feuille est lavée durant 30 mn à la température du laboratoire par du PBS + Triton X100 3% , puis trois fois 5 mn avec du PBS seul . La feuille est alors saturée avec du PBS contenant 5% de lait écrémé en poudre durant 1 h à 37°C , puis lavée avec du PBS + Tween 20 ( 0,2 % ) trois fois .

Une incubation est effectuée avec les immunsérums dilués au 1/20è dans le tampon PBS + Tween 20 ( 0,2% ) + lait en poudre 5% durant 1 h 30 à 37°C avec des agitations périodiques. Puis trois lavages en PBS + Tween sont effectués avant l'incubation avec les anticorps anti-immunoglobulines marqués avec la phosphatase alcaline . Les anticorps anti-immunoglobulines humains et anti-immunoglobulines de cobayes , marqués à la phosphatase (Biosys) sont utilisés à la dilution finale de 1/2500 en PBS + Tween 20 (0,2%) + lait (5%) . Après 1 h 30 d'incubation à 37°C, les feuilles de PVDF sont lavées trois fois en PBS + Tween, puis incubées à la température du laboratoire durant 5 à 10 mn, dans le tampon de révélation contenant BCIP et NBT ( Harlow et Lane , précédemment cités ). La réaction est arrêtée et après séchage des feuilles celles-ci sont photographiées.

### 6 ) Composition en acides aminés :

Une analyse de la composition globale en acides aminés est effectuée pour chaque fraction chromatographique dans l'unité de Chimie Organique de l'Institut Pasteur . Un analyseur Beckman LS 6300 est utilisé .

La composition globale exprimée en fréquence des acides aminés des protéines de 45-47 kD est la suivante :
ASN/ASP: 10,6% , THR : 5/4%; SER: 5%; GLN/GLU : 6%; GLY:7,4%; ALA : 19,2% ; VAL: 5,8%; ILE: 2,3%; LEU : 4,7%; TYR: 2,2%; PHE: 2,2% ; LYS : 2,9 % ; ARG: 2,5% ; PRO: 21,9%.

### EXEMPLE 2 :

### Détermination de la spécificité immunologique des protéines et fractions protéiques.

### A. Isolement des antigènes reconnus par les anticorps des cobayes immunisés avec des bacilles vivants.

Des groupes de 12 à 15 cobayes (femelles Hartley de 250 à 300 g au début de l'expérience ) ont reçu soit des mycobactéries vivantes ( 2 x 10⁷ unités viables de BCG en deux injections intradermiques dans 0,1 ml de solution saline ) , soit 2 mg de mycobactéries de la même souche tuées par la chaleur ( 120°C , 30 mn) en intramusculaire dans 0,5 ml d'une émulsion solution saline dans l'adjuvant de Freund incomplet (1/1) . Les sérums de différents groupes de cobayes ont été prélevés 7 à 12 mois après l'immunisation, filtrés ( 0,22 µm) puis distribués en petits volumes qui sont congelés et conservés à - 20°C . Des essais de plusieurs groupes d'immunsérums ont été effectués ( 5 après immunisation avec des bactéries vivantes et 6 après immunisation avec des bactéries tuées ) . Les résultats rapportés ont été obtenus avec un groupe de sérums représentatifs de chaque type d'immunisation; les différences entre groupes sont minimes pour le même schéma d'immunisation .

### 1°) Etape de filtration moléculaire sur Si 300.

Le milieu de culture ( lavé, concentré et lyophilisé) constituant le matériel de départ est injecté sous un volume d'échantillon de 10 ml contenant 500 mg de matériel sur la colonne Si 300. Les fractions 1 à 6 sont séparées selon le profil indiqué sur la figure 1 , regroupées pour les 24 injections successives , puis lavées , concentrées et lyophilisées . Le tableau 1 indique le poids brut de chaque fraction après lyophilisation ainsi que le poids minimum correspondant en protéines, calculé à partir des concentrations de chaque acide aminé classique déterminées par l'analyse en acides aminés de chaque fraction (analyseur Beckman LS 6300).

Chaque fraction (10 µg) est placée sur une piste d'un gel SDS ; puis , après la séquence électrophorèse, transfert sur membrane PVDF et immunodétection , les fractions contenant les protéines majoritaires réagissant avec les différents sérums sont repérées .

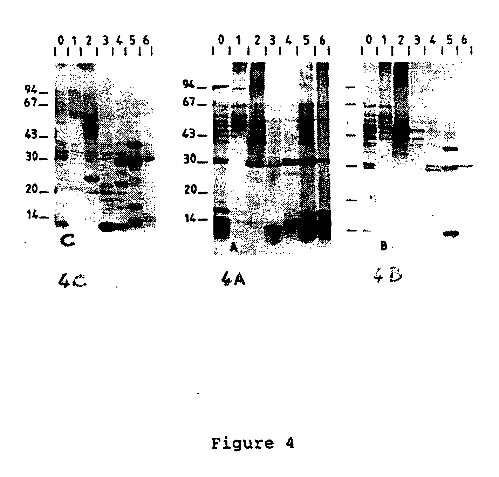

La figure 4 montre un gel coloré au bleu de Coomassie (Figure 4C) et deux immuno-empreintes de gels identiques révélées avec des sérums de cobayes immunisés avec des bacilles morts (4A) ou avec des bacilles vivants (4B) . Des antigènes communs sont reconnus par les deux types de sérums , tels que les antigènes de 30 kD présents dans les fractions 4, 5 et 6, et l'antigène de 38 kD dans la fraction 5. Des antigènes de poids moléculaire 10 à 16 kD dans les fractions 3, 4, 5 et 6 sont reconnus essentiellement par les anticorps des cobayes immunisés avec des bacilles morts . Deux antigènes de 45 et 47 kD présents dans la fraction 2 sont reconnus essentiellement par les anticorps des animaux immunisés avec des bacilles vivants . Cette fraction est sélectionnée pour la seconde étape de purification .

### 2) Etape sur colonne échangeuse d'ions :

Un échantillon de 100 mg de la fraction précédente est chargé sur une colonne DEAE-TSK préparative et éluées par un gradient de NaCl. Le profil à 220 nm des molécules éluées définit trois fractions principales ( figure 2). Après regroupement, chaque fraction obtenue par les injections successives du matériel est lavée, concentrée et lyophilisée (tableau 2).

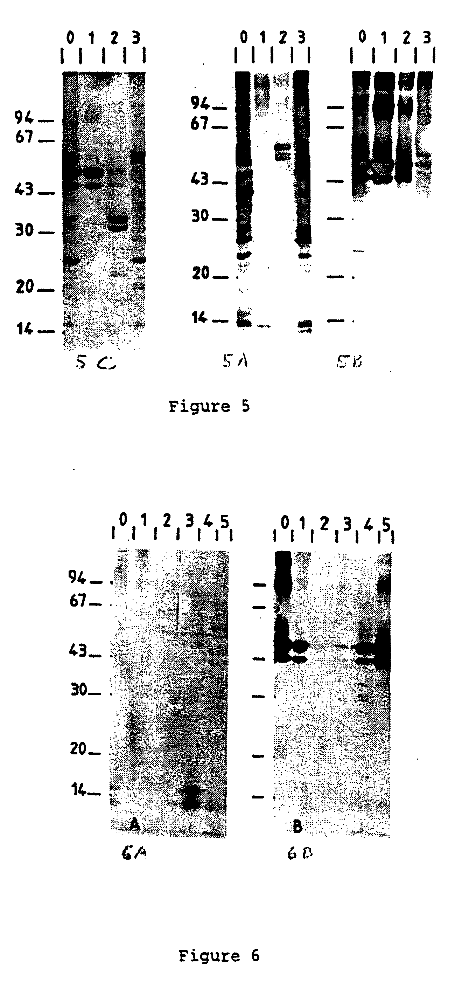

Après électrophorèse sur gel SDS de 5 µg de chacune des fractions précédentes, les immuno-empreintes sur feuille PVDF sont révélées par les sérums de cobayes immunisés avec des bacilles morts ou vivants (figures 5A et 5B ). La fraction 1-DEAE ne contient que quelques antigènes reconnus par les anticorps des animaux immunisés avec des bacilles morts: deux bandes faibles à 10 et 14 kD environ , une bande faible à 52 kD et une ombre mal définie au-dessus de 67 kD. Par contre, cette même fraction 1-DEAE contient un doublet à 45/47 kD fortement reconnu par les anticorps des cobayes immunisés avec des bacilles vivants, ainsi qu'une tache intense mal limitée entre 67 et 94 kD. Cette fraction 1-DEAE est choisie pour l'étape suivante de purification.

### 3) Etape sur colonne phase-inverse:

Une colonne RP 300 10µm , équilibrée avec le tampon acétate d'ammonium (20 mM) , reçoit un échantillon de 1 ml contenant 5 à 10 mg maximum de la fraction 1-DEAE précédente . L'élution avec un gradient d'acétonitrile de o à 90% selon le schéma de la figure 3 permet de récupérer cinq fractions principales . Ces fractions sont concentrées par évaporation sous vide à 40°C pour éliminer la majorité de l'acétonitrile, puis lyophilisées . Le tableau 3 indique les poids de chaque fraction.

La fraction 4 qui correspond à une élution entre 25 et 30% d'acétonitrile contient des antigènes de 10 à 15 kD reconnus par les anticorps présents dans le sérum des animaux immunisés avec des bacilles morts ainsi qu'un peu d'antigènes de 45/47 kD reconnus par les anticorps provenant des animaux immunisés avec des bacilles vivants . La fraction 5 suivante (gradient de 30 à 50% d'acétonitrile ) contient la majorité des molécules reconnues par les anticorps des animaux immunisés avec des bacilles vivants et essentiellement ces molécules ( figure 6).

### B) Essais des anticorps Drovenant de sujets atteints de tuberculose ou d'une autre maladie infectieuse.

1) Des sérums provenant de 14 malades, présentant une rechute de tuberculose pulmonaire ( 9 malades ) ou une première atteinte ( 5 malades ) , sont utilisés pour la caractérisation des antigènes principaux reconnus par l'homme lors d'une infection par M.tuberculosis.

| N° | Sexe | Age | |
|---|---|---|---|
| 77 | H | 33 | 3ème atteinte, tuberculose étendue |
| 104 | F | 47 | 2ème atteinte , tuberculose étendue |
| 105 | H | 49 | 2ème atteinte, tuberculose moyenne |
| 108 | H | 38 | 2ème atteinte, tuberculose minime sur des séquelles anciennes étendues |
| 115 | H | 64 | 2ème atteinte |
| 117 | H | 24 | 2ème atteinte |
| 124 | H | 63 | 2ème atteinte |
| 131 | H | 64 | 2ème atteinte, tuberculose actuelle très étendue |
| 134 | H | 33 | 3ème atteinte, tuberculose étendue |
| 123 | F | 26 | 1ère atteinte, tuberculose moyenne |
| 3A | M | 45 | 1ère atteinte, tuberculose étendue |
| 2G | F | 17 | 1ère atteinte, tuberculose moyenne |
| 2D | M | 27 | 1ère atteinte, tuberculose moyenne |
| 2A | M | 52 | 1ère atteinte, tuberculose étendue |

2 ) Des sérums, provenant de 13 malades atteints d'une maladie infectieuse sans histoire de tuberculose récente connue, sont utilisés pour la caractérisation des antigènes sans rapport direct avec l'infection par M.tuberculosis . Les sérums ont été prélevés pour établir ou confirmer les diagnostics de borréliose (5 cas), de leptospirose (3 cas) , de versiniose (2 cas ) ou de brucellose (3 cas).

Ces sérums de malades atteints de tuberculose ou d'une autre infection sont négatifs pour la présence d'anticorps anti-HIV et anti-Hbs ( antigène de surface du virus de l'hépatite B ) .

Les fractions obtenues après la première étape de séparation sur Si 300 ont été soumises à électrophorèse, puis au transfert sur membrane PVDF. Des membranes identiques ont été préparées et individuellement mises en présence d'un sérum provenant d'un sujet malade atteint de tuberculose ou d'une autre maladie infectieuse.

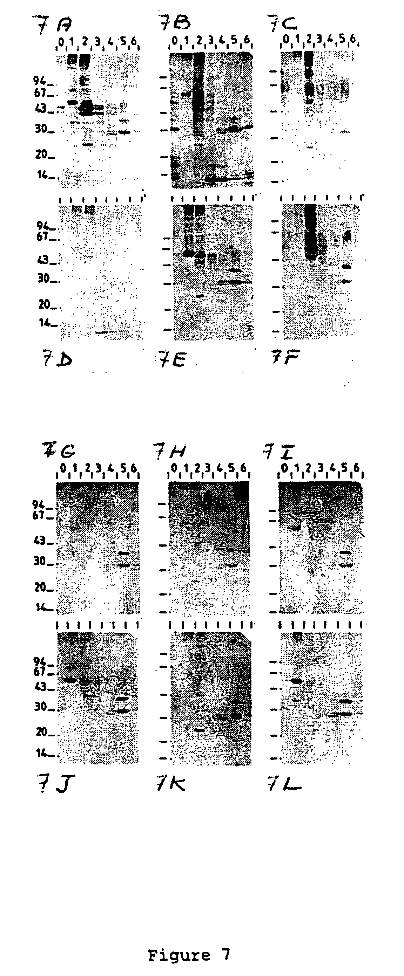

Les résultats obtenus pour 6 malades atteints de tuberculose et 6 malades atteints d'une autre infection montrent que des antigènes de 30 kD présents dans les fractions 4, 5 et 6 et des antigènes de 35/38 kD de la fraction 5 sont reconnus par tous les sérums . Dans la fraction 2 quelques antigènes , en particulier un antigène de 25 kD , sont aussi reconnus par tous les sérums . Par contre, seuls les sérums des malades atteints de tuberculose interagissent fortement avec des antigènes situés dans la zone 45/47 kD (figure 7).

Ces antigènes de 45/47 kD, purifiés comme indiqué plus haut , sont placés sur une piste très large d'un gel SDS, puis après électrophorèse, ils sont transférés sur une membrane PVDF qui est ensuite découpée en bandelettes de 3 mm environ . Chaque bandelette est incubée en présence d'un sérum de malades . Comme l'indique la figure 8, 12 des 14 sérums provenant de malades atteints de tuberculose reconnaissent les antigènes de 45/47 kD , alors qu'aucun des sérums de malades atteints d'autres infections ne reconnaît ces antigènes .

### C. Electrophorèse en deux dimensions des protéines du groupe 45-47 kD .

Une électrophorèse en deux dimensions des protéines du groupe de poids moléculaire 45-47 kD a été effectuée puis le gel est coloré à l'argent ( figure 9 ).

Les molécules sont ensuite transférées sur une feuille de PVDF puis mises en présence d'anticorps de cobayes immunisés avec des bacilles vivants ou avec des anticorps de cobayes immunisés avec des bacilles morts .

Les résultats de transfert montrent que les molécules colorées à l'argent sont détectées par les anticorps des cobayes immunisés avec des bacilles vivants , tandis qu'elles ne sont pas reconnues par les anticorps de cobayes immunisés avec des bacilles morts .

D'autre part , les molécules de 47 kD de ce complexe sont aussi reconnues par les anticorps monoclonaux de la lignée d'hybridomes déposée auprès de la CNCM sous le n°I. 1081.

### CONCLUSION:

Les résultats rapportés (figure 7 ) montrent qu'il existe dans une préparation de mycobactéries , ici dans un milieu de culture , des antigènes reconnus à la fois par les sérums de malades tuberculeux et par les sérums de malades atteints d'autres maladies infectieuses .

Par contre des antigènes situés dans la zone 45/47 kD et présents dans la fraction 2 du fractionnement sur colonne Si300 ne sont reconnus que par des sérums de malades atteints par la tuberculose et ne sont pas reconnus par des sérums de malades atteints d'une autre infection.

Les molécules de 45/47 kD, qui ont été purifiées sur leur capacité antigénique à réagir spécifiquement avec le sérum des cobayes immunisés avec des bacilles vivants, ont des pH isoélectriques compris entre 3,7 et 3,9, ainsi qu'on l'a déterminé sur gel d'immobilines .

En gel à deux dimensions, la bande à 47 kd se résout en deux taches principales après coloration au nitrate d'argent à des pHi de 3,7 et 3,9 , et la bande à 45 kD en une tache principale à pHi 3,7. Des taches de moyenne intensité sont aussi visualisées par cette technique, qui font partie du complexe 45/47 kD. Les différentes molécules mises ainsi en évidence en gel à deux dimensions sont toutes reconnues par le sérum des animaux immunisés avec des bacilles vivants .

Il n'existe pas d'autres molécules décelables après coloration du gel au bleu de Coomassie ou à l'argent .

De même , après transfert sur membrane PVDF puis immunodétection avec des sérums de cobayes immunisés avec des bacilles tués par la chaleur , il n'existe aucune tache visible dans la zone des antigènes 45/47 kD , ni ailleurs .

Des sérums de lapins immunisés contre une préparation brute d'antigènes de mycobactéries ne mettent en évidence que ces molécules de 45/47 kD , montrant ainsi leur pureté sur ces critères biochimiques et immunochimiques.

Un anticorps monoclonal préparé chez la souris reconnaît les différentes molécules de 47 kd dans un essai d'immunodétection après transfert sur membrane PVDF des molécules présentes sur un gel à deux dimensions.

Aucun des réactifs immunologiques qui ont été testés (sérums de cobayes après différentes durées d'immunisation par des bactéries vivantes, sérums de malades atteints de tuberculose ) n'a pu dissocier, sur la base de la réactivité antigénique, les molécules de ce complexe 45/47 kD.

La composition globale en acides aminés des protéines de ce complexe est aussi en faveur d'une parenté étroite entre elles .

**TABLEAU I -**

| Masse pondérale de chaque fraction Si 300 et évaluation du poids en protéines correspondant . | | | |
|---|---|---|---|
| Fraction | Poids brut (mg) | % en acides aminés | Poids en protéines (mg) |
| Fr 1 | 578 | 15 | 86 |
| Fr 2 | 23O | 64 | 147 |
| Fr 3 | 580 | 53 | 308 |
| Fr 4 | 460 | 51 | 236 |
| Fr 5 | 62 | 67 | 42 |
| Fr 6 | 370 | 44 | 161 |
| Total | 2280 | | 960 |
| Légende du tableau I. | | | |
| A partir de 12g de matériel brut , contenant au minimum 2,2 g de protéines , 6 fractions sont obtenues par filtration moléculaire sur Si 300. Le calcul du poids minimum correspondant en protéines est effectué à partir des résultats de la composition globale en acides aminés . | | | |
| Les rendements globaux sont de 19% pour le rendement brut pondéral et de 44% pour le rendement calculé en protéines. | | | |

**TABLEAU 2-**

| Masse pondérale de chaque fraction DEAE et évaluation du poids correspondant en protéines . | | | |
|---|---|---|---|
| Fraction | Poids brut (mg) | % en acides aminés | Poids en protéines (mg) |
| Fr 1 | 58,4 | 68 | 39,7 |
| Fr 2 | 8,4 | 32 | 2,7 |
| Fr 3 | 78,5 | 86 | 68,0 |
| Total | 145,3 | | |
| Légende du Tableau 2: | | | |
| La fraction 2 Si 300 précédente est chargée sur une colonne DEAE-TSK préparative . Une fraction non retenue par la colonne constitue la fraction 1-DEAE, les fractions 2 et 3 correspondent à l'élution par une force ionique croissante (entre 10mM et 600mM NaCl ) . | | | |
| Les rendements sont de 63% pour le rendement pondéral brut et de 75% pour le rendement calculé pour les protéines après analyse de la composition de chaque fraction en acides aminés. | | | |

**TABLEAU 3-**

| Masse pondérale de chaaue fraction phase inverse RP 300 et évaluation du poids correspondant en protéine . | | | |
|---|---|---|---|
| Fraction | Poids brut (mg) | % en acides aminés | Poids en protéines (mg) |
| Fr1 | 15,0 | 13 | 2,0 |
| Fr 2 | 2,3 | 18 | 0,4 |
| Fr 3 | 1,5 | 13 | 0,2 |
| Fr 4 | 4,1 | 65 | 2,7 |
| Fr 5 | 29,9 | 77 | 23,0 |
| Total | 52,8 | | 26,3 |
| Légende du Tableau 3: | | | |
| La fraction 1-DEAE précédente est chargée sur une colonne RP 300 (Aquapore ) , puis éluée avec un gradient d'acétonitrile de O à 90% selon le schéma indiqué figure 5. | | | |

## Revendications

1. Fraction protéique de Mycobacterium présentant un poids moléculaire compris entre environ 44,5 et environ 47,5 kD, tel que visualisé sur un gel d'électrophorèse de polyacrylamide en présence de SDS en conditions dénaturantes, ladite fraction protéique étant en outre **caractérisée en ce qu'**elle possède une composition en acides aminés exprimée en fréquence pour PRO d'environ 21,9% , pour ASN/ASP d'environ 10,6%, pour THR d'environ 5,4%, pour SER d'environ 5%, pour GLN/GLU d'environ 6%, pour GLY d'environ 7,4%, pour ALA d'environ 19,2%, pour VAL d'environ 5,8%, pour ILE d'environ 2,3%, pour LEU d'environ 4,7%, pour TYR d'environ 2,2%, pour PHE d'environ 2,2%, pour LYS d'environ 2,9%, et/ou pour ARG d'environ 2,5%.

2. Fraction protéique selon la revendication 1, **caractérisée en ce qu'**elle présente un poids moléculaire compris entre environ 45 et environ 47 kD, tel que visualisé sur un gel d'électrophorèse de polyacrylamide en présence de SDS en conditions dénaturantes.

3. Fraction protéique selon l'une des revendications 1 et 2, **caractérisée en ce qu'**elle est obtenue à partir de culture de Mycobacterium par un procédé comprenant au moins les étapes suivantes:
- élimination des bactéries du milieu de culture par filtration,
- passage du filtrat sur tamis moléculaire et répartition de l'éluat en fractions,
- sélection des fractions par détermination de leur réactivité vis-à-vis d'anticorps spécifiques de la tuberculose.

4. Fraction protéique selon la revendication 3, **caractérisée en ce que** le passage sur tamis moléculaire est suivi d'une chromatographie échangeuse d'ions.

5. Fraction protéique selon la revendication 4, **caractérisée en ce que** la chromatographie échangeuse d'ions est suivie d'une chromatographie en phase inverse.

6. Protéine constitutive de la fraction protéique selon l'une des revendications 1 à 5, **caractérisée en ce qu'**elle présente un poids moléculaire d'environ 45 kD, tel que visualisé sur un gel d'électrophorèse de polyacrylamide en présence de SDS en conditions dénaturantes, ainsi qu'un pHi d'environ 3,7.

7. Protéine constitutive de la fraction protéique selon l'une des revendications 1 à 5, **caractérisée en ce qu'**elle présent un poids moléculaire d'environ 47 kD, tel que visualisé sur un gel d'électrophorèse de polyacrylamide en présence de SDS en conditions dénaturantes, ainsi qu'un pHi d'environ 3,9.

8. Protéine selon la revendication 7, **caractérisée en ce qu'**elle est reconnue par un anticorps produit par la lignée d'hybridome déposée à la CNCM sous le n° I-1081.

9. Fraction protéique selon l'une des revendications 1 à 5 ou protéine selon l'une des revendications 6 à 8, **caractérisée en ce qu'**elle est reconnue par des anticorps obtenus par immunisation avec des bacilles de M.bovis vivants, par des anticorps de patients tuberculeux et n'est pas reconnue par des anticorps obtenus par immunisation avec des bacilles de M. bovis tués par la chaleur ou par des anticorps de patients sains ou atteints par une maladie autre que la tuberculose.

10. Milieu de culture **caractérisé en ce qu'**il comprend une fraction protéique selon l'une des revendications 1 à 5 ou protéine selon l'une des revendications 6 à 8.

11. Protéine hybride comprenant une des protéines selon l'une des revendications 6 à 8 et un déterminant antigénique provenant d'un agent biologique autre que M. bovis.

12. Lignée d'hybridomes déposée sous le n° I-1081 auprès de la CNCM.

13. Anticorps sécrété par la lignée d'hybridomes selon la revendication 12.

14. Utilisation d'une fraction protéique selon l'une des revendications 1 à 5 ou d'une protéine selon l'une des revendications 6 à 8 pour la détection *in vitro* d'anticorps reconnaissant ladite fraction protéique ou ladite protéine dans un échantillon provenant d'un patient.

15. Utilisation selon la revendication 13, **caractérisée en ce que** la détection in vitro est effectuée par des méthodes enzymoimmunologiques ou radioimmunologiques ou par des immunoempreintes.

16. Coffret de dosage pour la mise en oeuvre de l'utilisation selon l'une des revendications 14 et 15.

17. Vaccin ou médicament contenant au moins une protéine, une fraction protéique , une protéine ou un anticorps selon l'une des revendications 1 à 11 et 13.

18. Composition pharmaceutique contenant une quantité efficace d'une protéine, d'une fraction protéique ou d'un anticorps selon l'une des revendications 1 à 11 et 13 en association avec des diluants ou adjuvants pharmaceutiquement acceptables.

19. Utilisation des protéines, fractions protéiques ou anticorps selon l'une des revendications 1 à 11 et 13 pour la fabrication d'un médicament pour le traitement ou la prévention de la tuberculose.

## Claims

1. Protein fraction from Mycobacterium having a molecular weight between about 44.5 and 47.5 kD such as obtained on a polyacrylamide electrophoresis gel in the presence of SDS with denaturing conditions, said protein fraction is further **characterized in that** it has an amino-acid composition expressed by frequency for PRO of approximately 21.9 %, for ASN/ASP approximately 10.6 %, for THR approximately 5.4 %, for SER approximately 5 %, for GLN/GLU approximately 6 %, for GLY approximately 7.4 %, for ALA approximately 19.2 %, for VAL approximately 5.8 %, for ILE approximately 2.3 %, for LEU approximately 4.7 %, for TYR approximately 2.2 %, for PHE approximately 2.2 %, for LYS approximately 2.9 %, and/or for ARG approximately 2.5 %.

2. Protein fraction according to claim 1, **characterized in that** it has a molecular weight between about 45 and 47 kD such as obtained on a polyacrylamide electrophoresis gel in the presence of SDS with denaturing conditions.

3. Protein fraction according to one of claims 1 and 2, **characterized in that** it is obtained from a Mycobacterium culture by a process comprising at least the following steps :
- elimination of the bacteria from the culture medium by filtration,
- passage of the filtrate over a molecular sieve, and division of the eluate into fractions, and
- selection of the fractions by determination of their immunological reactivity towards specific tuberculosis antibodies.

4. Protein fraction according to claim 3, **characterized in that** the passage of the filtrate over a molecular sieve is followed by an ion exchange chromatography.

5. Protein fraction according to claim 4, **characterized in that** the ion exchange chromatography is followed by a reversed phase chromatography.

6. Protein constituting the protein fraction according to one of claims 1 to 5, **characterized in that** it has a molecular weight of 45 kD such as obtained on a polyacrylamide electrophoresis gel in the presence of SDS with denaturing conditions and a pH of about 3.7.

7. Protein constituting the protein fraction according to one of claims 1 to 5, **characterized in that** it has a molecular weight of about 47 kD such as obtained on a polyacrylamide electrophoresis gel in the presence of SDS with denaturing conditions and a pH of about 3.9.

8. Protein according to claim 7, **characterized in that** it is recognized by an antibody secreted by a hybridoma line deposited under N° I-1081 at the CNCM.

9. Protein fraction according to one of claims 1 to 5 or protein according to one of claims 6 to 8, **characterized in that** it is recognized by antibodies obtained by immunization with live M. bovis bacilli, by the antibodies from tubercular patients and is not recognized by the antibodies obtained by immunization with heat killed M. bovis bacilli or by the antibodies from healthy patients or patients affected by a disease other than tuberculosis.

10. Culture medium, **characterized in that** it comprises a protein fraction according to one of claims 1 to 5 or a protein according to one of claims 6 to 8.

11. Hybrid protein comprising one of the proteins according to one of claims 6 to 8 and an antigenic determinant from a biological agent other than M. bovis.

12. Hybridoma line deposited under N° I-1081 at the CNCM.

13. Antibody secreted by the hybridoma line according to claim 12.

14. Use of a protein fraction according to one of claims 1 to 5 or a protein according to one of claims 6 to 8 for the in vitro detection of antibodies recognizing said protein fraction or said protein in a sample from a patient.

15. Use according to claim 13, **characterized in that** the in vitro detection is made by enzymoimmunological or radioimmunological methods or by immunoprints.

16. Kit to carry over the use according to one of claims 14 and 15.

17. Vaccine or drug comprising at least a protein, a protein fraction, a protein or an antibody according to one of claims 1 to 11 and 13.

18. Pharmaceutical composition comprising an effective amount of a protein, a protein fraction or an antibody according to one of claims 1 to 11 and 13 in combination with diluents or suitable pharmaceutical vehicles.

19. Use of proteins, protein fractions or antibodies according to one of claims 1 to 11 and 13 for making a drug to cure or prevent tuberculosis.

## Patentansprüche

1. Proteinfraktion von *Mycobacterium* mit einem Molekulargewicht zwischen etwa 44,5 und etwa 47,5 kD, sichtbar gemacht auf einem Polyacrylamid-Elektrophorese-Gel in Gegenwart von SDS unter denaturierenden Bedingungen, wobei die Proteinfraktion außerdem **dadurch gekennzeichnet ist, dass** sie folgende als Häufigkeit ausgedrückte Aminosäurezusammensetzung hat: für Pro ungefähr 21,9%, für Asn/Asp ungefähr 10,6%, für Thr ungefähr 5,4%, für Ser ungefähr 5%, für Gln/Glu ungefähr 6%, für Gly ungefähr 7,4%, für Ala ungefähr 19,2%, für Val ungefähr 5,8%, für Ile ungefähr 2,3%, für Leu ungefähr 4,7%, für Tyr ungefähr 2,2%, für Phe ungefähr 2,2%, für Lys ungefähr 2,9% und/oder für Arg ungefähr 2,5%.

2. Proteinfraktion gemäß Anspruch 1, **dadurch gekennzeichnet, dass** sie ein Molekulargewicht zwischen etwa 45 und etwa 47 kD aufweist, sichtbar gemacht auf einem Polyacrylamid-Elektrophorese-Gel in Gegenwart von SDS unter denaturierenden Bedingungen.

3. Proteinfraktion gemäß einem der Ansprüche 1 und 2, **dadurch gekennzeichnet, dass** sie ausgehend von einer *Mycobacterium-*Kultur durch ein Verfahren erhalten wird, das wenigstens die folgenden Schritte umfasst:
- Entfernung der Bakterien aus dem Kulturmedium durch Filtration;
- Passieren des Filtrats über Molekularsieb und Verteilung des Eluats auf Fraktionen;
- Auswählen der Fraktionen durch Bestimmung ihrer Reaktivität gegenüber Tuberkuiose-spezifischen Antikörpern.

4. Proteinfraktion gemäß Anspruch 3, **dadurch gekennzeichnet, dass** sich an das Passieren über Molekularsieb eine Ionenaustauscherchromatographie anschließt.

5. Proteinfraktion gemäß Anspruch 4, **dadurch gekennzeichnet, dass** sich an die Ionenaustauscherchromatographie eine Umkehrphasenchromatographie anschließt.

6. Proteinbestandteil der Proteinfraktion gemäß einem der Ansprüche 1 bis 5, **dadurch gekennzeichnet, dass** er ein Molekulargewicht von ungefähr 45 kD, sichtbar gemacht auf einem Polyacrylamid-Elektrophorese-Gel in Gegenwart von SDS unter denaturierenden Bedingungen, sowie ein pHi von ungefähr 3,7 aufweist.

7. Proteinbestandteil der Proteinfraktion gemäß einem der Ansprüche 1 bis 5, **dadurch gekennzeichnet, dass** er ein Molekulargewicht von ungefähr 47 kD, sichtbar gemacht auf einem Polyacrylamid-Elektrophorese-Gel in Gegenwart von SDS unter denaturierenden Bedingungen, sowie ein pHi von ungefähr 3,9 aufweist.

8. Protein gemäß Anspruch 7, **dadurch gekennzeichnet, dass** es von einem Antikörper erkannt wird, der durch die Hybridomlinie erkannt wird, die beim CNCM unter der Nr. I-1081 hinterlegt wurde.

9. Proteinfraktion gemäß einem der Ansprüche 1 bis 5 oder Protein gemäß einem der Ansprüche 6 bis 8, **dadurch gekennzeichnet, dass** sie bzw. es von Antikörpern, die durch Immunisierung mit lebenden *M*.-*bovis*-Bazillen erhalten werden, und von Antikörpern von Tuberkulosepatienten erkannt wird und von Antikörpern, die durch Immunisierung mit durch Hitze abgetöteten *M*.-*bovis*-Bazillen erhalten werden, oder von Antikörpern von gesunden Patienten oder solchen, die unter einer anderen Krankheit als Tuberkulose leiden, nicht erkannt wird.

10. Kulturmedium, **dadurch gekennzeichnet, dass** es eine Proteinfraktion gemäß einem der Ansprüche 1 bis 5 oder ein Protein gemäß einem der Ansprüche 6 bis 8 umfasst.

11. Hybridprotein, das eines der Proteine gemäß einem der Ansprüche 6 bis 8 und eine antigene Determinante, die von einem anderen biologischen Agens als *M*. *bovis* stammt, umfasst.

12. Hybridomlinie, die beim CNCM unter der Nr. I-1081 hinterlegt wurde.

13. Antikörper, der von der Hybridomlinie gemäß Anspruch 12 sezerniert wird.

14. Verwendung einer Proteinfraktion gemäß einem der Ansprüche 1 bis 5 oder eines Proteins gemäß einem der Ansprüche 6 bis 8 für den in-vitro-Nachweis von Antikörpern, die die Proteinfraktion oder das Protein erkennen, in einer Probe, die von einem Patienten stammt.

15. Verwendung gemäß Anspruch 13, **dadurch gekennzeichnet, dass** der In-vitro-Nachweis mit enzymimmunologischen oder radioimmunologischen Verfahren oder durch Immunoblotting erfolgt.

16. Assaykit für die Durchführung der Verwendung gemäß einem der Ansprüche 14 und 15.

17. Impfstoff oder Medikament, das wenigstens ein Protein, eine Proteinfraktion, ein Protein oder einen Antikörper gemäß einem der Ansprüche 1 bis 11 und 13 enthält.

18. Pharmazeutische Zusammensetzung, die eine wirksame Menge eines Proteins, einer Proteinfraktion oder eines Antikörpers gemäß einem der Ansprüche 1 bis 11 und 13 in Verbindung mit pharmazeutisch annehmbaren Verdünnungsmitteln oder Adjuvantien enthält.

19. Verwendung der Proteine, Proteinfraktionen oder Antikörper gemäß einem der Ansprüche 1 bis 11 und 13 zur Herstellung eines Medikaments zur Behandlung oder Prävention der Tuberkulose.
